# EUROPEAN PATENT APPLICATION

(11) **EP 1 721 620 A1**
(43) Date of publication of application: **15.11.2006**
(21) Application number: 05708111.9
(22) Date of filing: 10.02.2005
(51) Int. Cl.: A61K 47/26, A61K 9/16

(54) **DIRECTLY-COMPRESSIBLE XYLITOL POWDER**

(30) Priority: 17.02.2004 ES 200400453
(71) Applicant: ENRIQUE BERNAT F., S.A., 08940 Cornella de Llobregat (ES)
(72) Inventor: LIZANO IGLESIAS, Joaquin, E-08635 SANT ESTEVE SESROVIRES (ES); FONT MESTRES, Joan Carles, E-08784 ELS HOSTALETS DE PIEROLA (ES)
(74) Representative: Barlocci, Anna
(86) International application number: PCT/ES2005/070014
(87) International publication number: WO 2005/082417

(57) **Abstract**

The invention relates to a directly-compressible granular xylitol powder comprising xylitol as the main component thereof and dextrin in a proportion of between 1 and 10 % by dry weight. In particular, the aforementioned dextrin is present in a proportion of 3.5 % by dry weight and comprises 85 % soluble fibre by dry weight. The tablets produced from the xylitol powder display a high degree of hardness, low friability and excellent palatability. In addition, said tablets maintain the desired sweetness as well as all of the healthy properties of xylitol, providing the prebiotic effect of the fibre.

## Description

### Directly compressible powder of xylitol

This invention relates to compositions for the manufacture of tablets in the field of the food, cosmetic and pharmaceutical industry.

### BACKGROUND ART

Saccharose is the most common sweetener used in the pharmaceutical and food industry. Nevertheless, excessive sugar consumption relates to a number of physiological alterations, such as obesity and the development of caries. Thus, the present tendencies are about to reduce excessive sugar consumption.

Some products are commercially available as saccharose substitutes, with a lower caloric value and further anti-cariogenic properties. Xylitol is preferred among them because of its excellent properties. Xylitol (formula I) is a natural alcohol and it has a simmilar sweetness strenght to saccharose, but a lower caloric content. Xylitol diminishes incidence of caries, impairing the adhesion of the bacteries to the dental plaque, it contributes to teeth re-mineralitzation, and even reverts incipient caries (cfr. K.K. Makinen et al., "Xylitol chewing gums and caries rates: a 40-month cohort study", J. Dent. Res. 1995, vol. 74, p. 1904-13; T. Kontiokari et al., "Antiadhesive effects of xylitol on otopathogenic bacteria", J. Antimicrob. Chemother. 1998, vol. 41, p. 563-5; W.H. Bowen et al., "The effects of sucralose, xylitol, and sorbitol on remineralization of caries lesions in rats", J. Dent. Res. 1992, vol. 71, p. 166-8). Xylitol is also used for the treatment of certain types of otitis and for the improvement of intestinal absorption of calcium. The final product has a very pleasant refreshing effect inside the mouth (cooling effect) thanks to the lower fusion enthalpy, i.e. very negative, of xylitol. 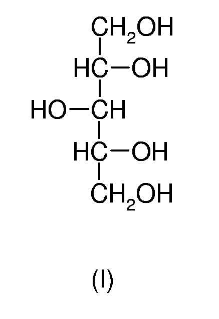 Xylitol, among other uses, is a constituent of tablets in the pharmaceutical, cosmetical and food industries. In the pharmaceutical industry, the tablets are used for administering active ingredients with a suitable size, shape and texture (e.g. for chewing, sucking, swallowing or dissolving). Xylitol is used for the preparation of sweet tablets in the food industry, especially in confection. Xylitol is excellent also for mouth and dental healthcare products because of its refreshing and anti-cariogenic effect.

Pills of xylitol are usually made by compression or direct molding of the powder which has been previously mixed and prepared for the process. Some conditions have to be fulfiled to be able to use a powder in the modern high-production machinery. One condition to fulfil is that the mixture of powders to be compressed has to be fluent enough to fill correctly the molding die. Furthermore, the mixture of the ingredients have to be homogeneous to maintain the right proportion of ingredients in every pill. Nevertheless, xylitol do not have the suitable conditions to be compressed. It has an insufficient flowing rate for working with high-production machinery and it does not mixes correctly, because it tends to form clots. Pills manufactured from xylitol have very low cohesion and compressibility, whether a crystalline or a milled and sifted xylitol is used, and the prepared pills break up easily and they do not fulfill marketing standards.

The previous granulation of the powder, optionally followed by a drying step, is usually used to make the powder materials suitable to be compressed. Generally, granulation comprises a physical mixing treatment wherein a certain binder is added. Some binders have been proposed to improve the properties of the granulated powder of xylitol when added in the granulation step. A first attemp is based in the addition of polyols such as sorbitol, mannitol or lactitol (see US 5.958.471; US 2003/0039684 and ES 2.041.346). Although, poliols (also classified as hydrogenated starch hydrolisate products) adversely affect the stability against humidity of the final products and other agents of tablet degradation, and sometimes they also adversely affect the palatability of the final product. Methods for the granulation of xylitol with carboxymethylcellulose (CMC) and polydextroses (see, US 5.204.115) has also been proposed. These products have serious disadvantages. At first, the amount of added CMC needed for these uses can exceed the maximmum levels allowed by certain legislations. Furthermore, solutions of CMC greater than 10% have viscosities above 65000 cp (centipoises), therefore they can be only used for batch production, without the possibility of being used for continuous production. Furthermore, polydextroses also have serious limitations in said uses. One of the most important limitations is that the hardness values of the tablets are in the lower limit to use them in the industrial compression machinery without breaking up the resulting tablets. Furthermore, although polydextroses are very water-soluble (up to 70-80%), the viscosity of the solution is very high impairing the intimate mixture of the ingredients in the granulation mill. At last, polydextroses are hygroscopic like CMC, so that the stability of the obtained tablets is compromised. Because of the previously cited properties of hygroscopicity and viscosity, CMC and polydextroses are more suitable for other uses such as the manufacturing of ice-creams, sauces, creams and mermelades.

So, It can be deduced from the state of the art that it is desirable to provide new binder agents for the granulation and the later compression of compositions based in xylitol, which overcome some of the serious limitations cited before and show additional advantadgeous effects.

### SUMMARY OF THE INVENTION

The inventors have surprisingly found that the use of dextrine as a binder for the granulation of xylitol allows to fulfill the suitable properties of hardness, friability and palatability of the tablets manufactured from these granulates.

So, the present invention provides a granulated and directly compressible powder of xylitol which comprises xilytol as main ingredient and dextrine, wherein dextrine is in a ratio of 1-10% by dry weight. In a embodiment of the invention, the dextrine is in a ratio of 3-5% by dry weight, and more preferably of 3.5% by dry weight.

The dextrine is a product formed through the process of the cleavage of starch into glucose. The name for dextrose refers to the fact that its solutions are intensely dextrorotatory in polarimetric assays, i.e. it causes polarized light to rotate to the right. The preferred dextrines of the invention are those from wheat or corn with a high content of soluble dietary fiber and a polimeritzation rate from 12 to 25. As an embodiment of the invention, dextrine comprises a 60-95% by dry weight of soluble fiber. As a further embodiment of the invention, dextrine comprises a 80-95% by dry weight of soluble dietary fiber, and more preferably a 85% by dry weight of soluble dietary fiber. A preferred dextrine for the present invention is composed of branched chains of glucose with a number of monomers between 12 and 25, wherein glycosidic bonds have the following proportions: 41% of alpha(1-4), 32% of alpha(1-6), 13% of alpha(1-2) and 14% of alpha(1-3).

Dietary fiber is the fraction of food which is not digested by the organism because it lacks of suitable enzymes for dietary fiber degradation. Dietary fiber is classified as soluble or insoluble dietary fiber, according to the capability of the fiber to be dissolved in water. Soluble dietary fiber is naturally found in a number of cereals, fruits and legumes. It has been proved that the comsumption of dietary fiber reduces cholesterol and LDL blood levels (Low Density Lipoproteins). Furthermore, dietary fiber has the capability of retaining water, increasing the size of the feces and easing bowel movement. Thus, a granulated and directly compressible powder of xylitol is obtained using dextrine as a binder with the healthy properties provided by the dietary fiber.

The preferred xylitol to conform the granulate is a milled and sifted xylitol with a particle size lower than 120 microns, but it can be used different particle sizes to obtain slightly different granulates.

In addition to xylitol and dextrine, the granulated and directly compressible powder of xylitol of the present invention can contain intensive sweeteners or mixtures of intensive sweeteners, such as aspartame, acesulfame K, saccharine and salts thereof, cyclamate and sats thereof, neohesperidine dihydrochalcone, thaumatin, neotame and sucralose. In another embodiment of the invention, at least one nutrient is added to the granulated powder of xylitol, selected from the group consisting in vitamines, minerals, anthocyans, polyphenols, protein of soya, vegetal extracts and aminoacids. In another embodiment, at least one dietary or cosmetic color is added, both authorised by applicable legislation. In another embodiment of the invention, at least a nutrient considered as prebiotic and/or probiotic is added. At last, as another embodiment, at least an active pharmaceutical and/or an active cosmetic ingredient is added (active ingredients for mouth and teeth healthcare are considered in the present invention as cosmetics). So that it is intended to obtain a granulated and directly compressible powder of xylitol with the maximmum amount of xylitol, mantaining the optimmum properties of hardness, friability and palatability of the obtained tablets.

The granulation of the powder of xylitol of the present invention is performed according to the methods of conventional granulation know by the person skilled in the art.

The present invention relates also to the preparation of tablets or pills by compression of the granulated and directly compressible powder of xylitol described before. The tablets can be chewable, effervescent, coated, prepared as retard delivery formulation, multi-layered, etc. The tablets are directly obtained by the compression of the previously mixed and prepared powder. The compression can be carried out in three steps: filling of the molding die, the compression (simple or double), and the ejection of the tablet. The correct filling of the dies depends on the correct flow of powder to compress and the speed of the tablet machine. The compression parameters are adjusted depending on the weight and the diameter of the tablet, whereas the ejection needs of the correct lubrication of the powder mixture, generally by the addition of the suitable amount of a lubricant.

The granulated and directly compressible powder of xylitol of the invention has very suitable properties for compression. The density of solutions up to 60% by weight of dextrine with a high content of soluble dietary fiber is low enough to allow a correct spraying over xylitol in the granulating mill. Thus, a nearly perfect mixture is obtained in the mill. Furthermore, the flowing properties in the tablet machine are improved. Tablets manufactured from the powder achieve appreciable higher values on hardness and lower on friability. The tablets mantain the desired sweetness, all the healthy properties of xylitol, and the prebiotic effect of the dietary fiber.

Throughout this description the term "hardness" relates to the force that the tablet can support before breaking up. The term "friability" relates to the amount of powder removed by friction. A binder is understood as a material capable of binding pieces of one or more substances and giving cohesion to the whole solely by physical effects. Throughout this description, the terms tablet and pill are used with the same meaning.

Throughout the description and claims the word "comprise" and variations of the word, such as "comprising", is not intended to exclude other technical features, additives, components, or steps. The content of the abstract of the present application, are incorporated herein as reference. Additional objects, advantages and features of the invention will become apparent to those skilled in the art upon examination of the description or may be learned by practice of the invention. The following examples and drawings are provided by way of illustration, and is not intended to be limiting of the present invention.

### DETAILED DESCRIPTION OF PARTICULAR EMBODIMENTS

The xylitol used to make the granulate is a milled and sifted xylitol with a particle size lower than 120 microns. The binder used is Nutriose^{®} FB (Roquette) dextrine, with a high content on soluble dietary fiber (85% of soluble dietary fiber according to the AOAC official method 2001.03).

To hardness measurement, an Erweka TBH 20 hardness tester was used which give the number of kp needed to break up the triangular tablets with 7.5 mm of diameter used in the experiments. When a milled or a crystallized xylitol is compressed, the hardness of the tablet do not exceed 2-3 kp, far away from the minimmum value required to the suitable handling of the tablets.

### Preparation in the laboratory

10 grams of a 60% by weight solution in water of Nutriose^{®} FB were added to 100 g of milled xylitol in a mixer with asymmetric and superposed blades stirring at 3200 rpm for 15 seconds. The obtained mixture was sifted through a screen with a mesh of 1.0 mm, and it was used to compress tablets of 7.5 mm of diameter and 200 mg of weight, with a main compression force of 20 kN.

The experiment was repeated adding various quantities of Nutriose solution to the xylitol. The results of the amount of dextrine solution added as final % of soluble dietary fiber are shown in TABLE 1. In all cases, palability and freshness of the obtained tablets was considered as optimum.

**TABLE 1: Results of the experiment at the laboratory**

| Content on soluble fiber | Hardness |
|---|---|
| 4.8% | 9.8% |
| 3.6% | 8.2% |
| 3.0% | 8.4% |
| 1.5% | 5.6% |

### Industrial manufacturing

Powder of xylitol was used in a vertical mixer to granulate the mixture continuosly, with a flow of 1000 kg/h. The 60% solution of Nutriose^{®} FB was injected at 100 l/h over a mixer stirring at 3200 rpm. The obtained product was sifted before drying in a four step furnace at 60 ºC, 45 ºC, and between 20 ºC and 25 ºC in the last two steps. The obtained product was sifted and its humidity resulted below to 0.5%, whereas its apparent density was 0.72-0.76 g/ml. The obtained flow was suitable for high production machinery.

A typical compression mixture was prepared with the obtained product. The results of the hardness test were simmilar to those for the product prepared in the laboratory. The composition of the mixture was the following:

| | |
|---|---|
| Powder of xylitol with a dry 3.5% of dextrine | 88.4% |
| Powder of mint aroma | 10.0% |
| Lubricant (Powder of hydrogenated vegetal oil) | 1.0% |
| Aspartame | 0.6% |

20 tablets of the same composition were tested resulting in an average hardness of 9.83 kp (95% of the tablets had a hardness comprised between 9.26 kp and 10.39 kp). The tablets manufactured by this process had a suitable palatability, friability, weight, calibre and shape.

The obtained granulate had an excellent flowing rate and compressibility using an estandard high production compression equipment, such as Fette P3090. Tablets of 7.5 mm of diameter, 4.5 mm of height and 200 mg of weight were obtained with a productivity of 350,000 units/hour.

## Claims

1. Granulated and directly compressible powder of xylitol which comprises xilytol as mean ingredient and dextrine, wherein dextrine is in a ratio of 1-10% by dry weight.

2. Powder of xylitol according to claim 1, wherein dextrine is in a ratio of 3-5% by dry weight.

3. Powder of xylitol according to claim 2, wherein dextrine is in a ratio of 3.5% by dry weight.

4. Powder of xylitol according to any one of claims 1-3, wherein dextrine comprises a ratio of 60-95% by dry weight of soluble dietary fiber.

5. Powder of xylitol according to claim 4, wherein dextrine comprises a ratio of 80-90% by dry weight of soluble dietary fiber.

6. Powder of xylitol according to claim 5, wherein dextrine comprises a ratio of 85% by dry weight of soluble dietary fiber.

7. Powder of xylitol according to any one of claims 1-6, further comprising at least an intensive sweetener.

8. Powder of xylitol according to claim 7, wherein the intensive sweeteners are selected from the group consisting of aspartame, acesulfame K, saccharin and salts thereof, cyclamate and salts thereof, neohesperidin dihydrochalcone, thaumatin, neotame and sucralose.

9. Powder of xylitol according to any one of claims 1-8, further comprising at least a nutrient selected from the group consisting of vitamines, minerals, anthocyans, polyphenols, protein of soya, vegetal extracts and aminoacids.

10. Powder of xylitol according to any one of claims 1-9, further comprising at least a dye.

11. Powder of xylitol according to any one of claims 1-10, further comprising at least a nutrient considered as prebiotic and/or probiotic.

12. Powder of xylitol according to any one of claims 1-11, further comprising at least an active pharmaceutical ingredient and/or an active cosmetic ingredient.

13. Tablet resulting from the compression of powder of xylitol according to any one of the preceding claims, and suitable excipients or adjuvants.
